(19) Europäisches Patentamt
European Patent Office
Office européen des brevets

(11) **EP 1 368 022 B1**

(12) **FASCICULE DE BREVET EUROPEEN**

(45) Date de publication et mention
de la délivrance du brevet:
**20.06.2007 Bulletin 2007/25**

(21) Numéro de dépôt: **02700095.9**

(22) Date de dépôt: **04.03.2002**

(51) Int Cl.:
**A61K 31/28** *(2006.01)*     **A61K 9/08** *(2006.01)*

(86) Numéro de dépôt international:
**PCT/CH2002/000133**

(87) Numéro de publication internationale:
**WO 2002/069959 (12.09.2002 Gazette 2002/37)**

(54) **UTILISATION D'UN FLACON CONTENANT UNE SOLUTION D'OXALIPLATINE**

VERWENDUNG EINES EINE OXALIPLATINLÖSUNG BEINHALTENDEN FLÄSCHCHENS

USE OF A VIAL CONTAINING AN OXALIPLATINUM SOLUTION

(84) Etats contractants désignés:
**AT BE CH CY DE DK ES FI FR GB GR IE IT LI LU
MC NL PT SE TR**
Etats d'extension désignés:
**AL LT LV MK RO SI**

(30) Priorité: **02.03.2001 CH 389012001**

(43) Date de publication de la demande:
**10.12.2003 Bulletin 2003/50**

(73) Titulaire: **DEBIOPHARM S.A.**
**1002 Lausanne (CH)**

(72) Inventeurs:
• **IBRAHIM, Houssam**
**CH-1255 Veyrier (CH)**
• **BLUNDELL, Ross**
**F-34980 Montferrier sur Lez (FR)**
• **GRAY, Martin**
**Alnwick**
**Northumberland NE66 2NJ (GB)**

(74) Mandataire: **Besse, François**
**ANDRE ROLAND S.A.**
**Avenue Tissot 15**
**P.O. Box 1255**
**1001 Lausanne (CH)**

(56) Documents cités:
**EP-A- 0 180 286**     **US-A- 4 566 600**

EP 1 368 022 B1

**Description**

La présente invention concerne l'utilisation d'un flacon contenant une préparation pharmaceutique d'oxaliplatine en solution aqueuse .

L'oxaliplatine (DCI; appelé également /-OHP), dérivé complexe du platine (CAS RN: 61825-94-3) décrit par Kidani et al. dans J. Med. Chem., 1978, 21, 1315, est un agent antinéoplasique utilisé par voie intraveineuse tout particulièrement dans le traitement des cancers colorectaux métastatiques. Actuellement, il est utilisé en milieu hospitalier sous une forme lyophilisée et sa préparation liquide est reconstituée juste avant son administration qui s'effectue généralement en perfusion de courte durée.

L'oxaliplatine sous forme lyophilisée, est formulée avec une quantité importante de lactose (d'un facteur 9 en poids par rapport à l'oxaliplatine). C'est alors une poudre ou un gâteau de couleur blanchâtre. Lors de sa reconstitution, il est préconisé d'utiliser une quantité, soit de solution glucosée, soit d'une eau de qualité dite "préparation pour injection" (PPI), telle que la concentration en oxaliplatine dans la préparation ainsi obtenue soit d'environ 5,0 mg/ml.

Récemment, une préparation d'oxaliplatine pharmaceutiquement stable, prête à être administrée par voie parentérale en perfusion, constituée par une solution aqueuse d'oxaliplatine à une concentration d'environ 2 mg/ml, et ne contenant pas d'autres adjuvants, a été décrite par Ibrahim et al. dans WO 96/04904. Il y est préconisé de conserver une telle préparation liquide dans un flacon en verre neutre pour usage pharmaceutique.

Cette préparation offre au personnel hospitalier le grand avantage, d'une part, de ne plus à avoir à manipuler un certain nombre de flacons contenant, soit une poudre ou un gâteau cytotoxique, soit les solvants appropriés, lors de la reconstitution de la préparation pharmaceutique et, d'autre part, d'éviter tout risque d'utiliser par erreur une solution de reconstitution contenant des ions chlorure, telle qu'une solution de chlorure de sodium habituellement utilisée dans ce genre d'opération, qui a pour conséquence grave de dégrader la substance active.

Des préparations liquides d'oxaliplatine telles que celles décrites ci-dessus peuvent également être conservées dans des poches souples pour perfusion. Mauvernay a précisé dans WO 00/21527 qu'aucune dégradation n'avait alors été observée pendant une période d'au moins un an, ceci à la condition d'utiliser, comme matériaux plastique particulier se trouvant au contact direct de la préparation liquide d'oxaliplatine, un matériau exempt de polychlorure de vinyle (PVC).

De leur coté, Anderson et al. ont observé une certaine tendance de ces mêmes solutions aqueuses à se dégrader au cours du temps. Pour remédier à ce phénomène, ils ont proposé dans WO 99/43355 d'apporter à ces solutions une certaine quantité d'un agent stabilisant tel que l'acide oxalique et ils ont préconisé de conserver les préparations ainsi obtenues dans des récipients scellés tels que des ampoules, des seringues ou des poches souples de perfusion. Cette proposition n'est toutefois pas totalement satisfaisante du fait d'une certaine toxicité généralement attribuée à l'acide oxalique (voir The Merck Index, 11 ème édition, 1989, page 1093).

Les autorités de santé attachent un très grande importance à ce que les préparations pharmaceutiques ne soient administrées à des patients qu'avec un minimum d'effets secondaires qui pourraient même s'avérer néfastes pour la santé du patient. C'est ainsi qu'elles exigent, lorsque que, dans une préparation pharmaceutique, la ou les substances actives se trouvent en présence de certains sous-produits ou produits de dégradation, que leur soit démontrer par de long et fastidieux essais de toxicité que ces sous-produits n'ont pas d'action délétère.

Généralement, elles tolèrent, dans une préparation pharmaceutique contenant une substance active devant être administrée à un patient à raison d'une dose quotidienne comprise entre 100 mg et 2 g, la présence d'impuretés non caractérisées seulement si chacune de ces impuretés ne dépasse pas une quantité d'environ 0,2%, en poids par rapport au poids de la substance active.

A titre indicatif et dans le cas d'un traitement d'un patient par administration d'oxaliplatine, la posologie généralement recommandée lors d'un traitement mettant en oeuvre une perfusion courte durant entre 2 et 6 heures est comprise entre environ 85 mg et environ 130 mg d'oxaliplatine par $m^2$ de surface corporelle. Ceci revient, en prenant comme surface corporelle moyenne une valeur de 1,7 $m^2$, à administrer quotidiennement une dose comprise entre environ 145 mg et environ 220 mg d'oxaliplatine.

En partant de ces doses à administrer mentionnées ci-dessus et en considérant le nombre total et les quantités respectives de produits de dégradation présents dans une préparation pharmaceutique contenant de l'oxaliplatine, le taux d'impuretés total mesuré ne devrait pas dépasser 2,0% en poids par rapport au poids d'oxaliplatine après conservation sur une durée d'au moins 10 mois.

Il y avait donc un besoin de trouver de nouveaux remèdes à ces dégradations observées à long terme lorsqu'une préparation d'oxaliplatine en solution dans l'eau doit être conservée dans des flacons en verre, de tels remèdes devant, d'une part, ne mettre en oeuvre que des flaconnages en matériaux communément disponible dans le commerce et, d'autre part, exclure l'emploi de stabilisants chimiques qui pourraient s'avérer présenter une action délétère.

A cet effet, la présente invention a pour objet la mise à disposition d'un ensemble constitué, d'une part, d'une préparation pharmaceutique d'oxaliplatine en solution aqueuse et, d'autre part, d'un flacon en verre contenant ladite préparation, ladite préparation devant satisfaire entre autre, à l'échelle d'une durée de conservation d'au moins 10 mois, aux critères de pureté et/ou de stabilité mentionnés précédemment.

Ledit flacon est constitué d'un verre habituellement utilisé pour conserver des préparations pharmaceutiques liquides à usage parentéral. Il peut être obtenu selon un procédé dit "pressé-soufflé", soit un procédé dit "souflé-soufflé". De préférence, le verre choisi est un verre dit de type I comme défini par les pharmacopées américaine (United States Pharmacopeia 25- NF 20, 2002) et européenne (Pharmacopée Européenne, 4ème édition, 2002). De préférence encore, c'est un verre non tinté dit clair ou incolore. Un verre de type II comme défini par ces mêmes pharmacopées peut également être utilisé.

Ce type de verre est particulièrement recommandé pour sa résistance chimique, en particulier sa résistance hydrolytique, et sa très haute durabilité chimique. Il convient tout particulièrement pour être au contact de préparations pharmaceutiques tout aussi bien acides, neutres ou alcalines.

Ce type de verre est à base de borosilicate. Plus précisément, et à titre d'exemple, la composition chimique, exprimée en pourcentage pondéral, de quelques verres de type 1 trouvés dans le commerce est donnée dans le Tableau 1 (extrait de Technical Methods Bulletin No.3, Glass containers for small volume parenteral products: Factors for selection and test methods for identification, Parenteral drug association, 1982).

Tableau 1

| Composition chimique | Désignations commerciales des verres de type I | | | | | | |
|---|---|---|---|---|---|---|---|
| | Kimble KG-33 | Kimble KG-35 | Kimble N51 A | Wheaton NS-33 | Wheaton NS-51 | Wheaton NSV | Wheaton Type I Flint |
| $SiO_2$ | 80 | 69 | 71 | 81 | 73 | 73 | 70 |
| $B_2O_3$ | 13 | 13 | 11 | 13 | 10 | 10 | 10 |
| $Al_2O_3$ | 3 | 6 | 7 | 2 | 6 | 6 | 6 |
| $Fe_2O_3$ | 0 | 0 | 0 | 0 | 0 | 0 | 0 |
| ZnO | 0 | 0 | 0 | 0 | 0 | 0 | 0.5 |
| $TiO_2$ | 0 | 0 | 0 | 0 | 0 | 0 | 0 |
| MnO | 0 | 0 | 0 | 0 | 0 | 0 | 0 |
| BaO | 0 | 2 | 2 | 0 | 2 | 2 | 2 |
| CaO | 0 | 1 | 1 | 0 | 1 | 0,5 | 1 |
| MgO | 0 | 0 | 0 | 0 | 0 | 0 | 0.5 |
| $Na_2O$ | 4 | 8 | 6 | 4 | 6 | 7 | 9 |
| $K_2O$ | 0 | 1 | 2 | 0 | 1 | 1 | 1 |

Comme il peut être constaté, ce tableau laisse penser qu'aucun des constituants entrant dans la composition du verre ne devrait interférer chimiquement avec le complexe organométallique du platine présent dans la solution.

Malgré cela, la Demanderesse a observé, comme l'avaient précédemment fait Anderson et al., que des dégradations importantes se produisaient parfois.

Dans le cas présent, les préparations d'oxaliplatine en solution aqueuse dans lesquelles se produisaient ces dégradations étaient pourtant conservées pendant quelques mois à la température ambiante du laboratoire dans des flacons en verre, en particulier dans des flacons en verre de type I.

A l'issue de nombreuses études de stabilité de préparations d'oxaliplatine en solution aqueuse ne contenant pas d'agent stabilisant, comme peut l'être par exemple l'acide oxalique, et contenues dans différentes conditions de flaconnage, la Demanderesse a pu constater, de façon surprenante, que la stabilité de ces préparations dépendait de la géométrie des flacons.

Plus précisément, elle a pu montrer, qu'en utilisant des flacons en verre de formes différentes, et pour chacune des formes, de contenances différentes, l'existence d'une relation entre, d'une part, le rapport "Surface de contact de la solution aqueuse d'oxaliplatine avec un flacon d'une certaine contenance / Volume de remplissage dudit flacon par ladite solution d'oxaliplatine" et, d'autre part, le degré de stabilité de ladite solution d'oxaliplatine, degré de stabilité caractérisé par la mesure du taux d'impuretés totales présentes dans les différentes préparations pharmaceutiques contenues et conservées dans les différents flacons.

Dans la suite de la présente demande, le terme "surface" désignera la surface de contact de la solution aqueuse d'oxaliplatine avec un flacon en verre d'une certaine contenance et sera exprimée en $mm^2$, le terme "volume" désignera le volume de remplissage dudit flacon par ladite solution d'oxaliplatine et sera exprimé en $mm^3$.

L'ensemble, selon la présente invention, constitué, d'une part, d'une préparation pharmaceutique d'oxaliplatine en solution aqueuse et, d'autre part, d'un flacon en verre contenant ladite préparation se caractérise par le fait que le rapport Surface/Volume est inférieur à 0,26. De préférence, le rapport Surface/Volume est inférieur à 0,20.

Par ailleurs, la Demanderesse a pu déterminer que le rapport SurfaceNolume suivait la relation suivante :

$$R_0 + A.c.I_{max}$$

où

$R_0$ représente le rapport Surface/Volume maximal théorique pour lequel aucune impureté ne serait quantifiable (c'est à dire pour $I_{max} = 0\%$) en mettant en oeuvre techniques d'analyse habituellement recommandée par la pharmacopée;

A étant une constante exprimée en ml/(mg.mm);

c représentant la concentration en oxaliplatine exprimée en mg/ml; et

$I_{max}$ représentant le taux pondéral maximal d'impuretés totales non caractérisé admis.

L'invention sera décrite plus précisément à l'aide des l'exemples suivants et du dessin dans lequel:

- la Figure 1 représente le taux pondéral d'impuretés totales non caractérisées dans une préparation aqueuse d'oxaliplatine à une concentration de 5 mg/ml après 4 mois de conservation en fonction du rapport Surface/Volume;
- la Figure 2 représente le taux pondéral d'impuretés totales non caractérisées dans une préparation aqueuse d'oxaliplatine à une concentration de 7 mg/ml après 4 mois de conservation en fonction du rapport Surface/Volume;
- la Figure 3 montre une superposition des courbes illustrées sur les figures 1 et 2;
- la Figure 4 représente le taux pondéral d'impuretés totales non caractérisées dans une préparation aqueuse d'oxaliplatine à une concentration de 5 mg/ml après 1 mois de conservation en fonction du rapport Surface/Volume;
- la Figure 5 représente le taux pondéral d'impuretés totales non caractérisées dans une préparation aqueuse d'oxaliplatine à une concentration de 5 mg/ml après 5,5 mois de conservation en fonction du rapport Surface/Volume; et
- la Figure 6 représente le taux pondéral d'impuretés totales non caractérisées dans une préparation aqueuse d'oxaliplatine à une concentration de 5 mg/ml après 10 mois de conservation en fonction du rapport Surface/Volume.

1: Préparation et conservation des échantillons

Pour conduire cet essai, quatre séries de flacons, constitués d'un verre incolore de type I, tous de forme cylindrique mais de volumes différents ont été utilisés. Le Tableau 2 rassemble, pour chaque série de flacons, respectivement leur contenance dite "utile", leur contenance dite "ras-bord", le diamètre intérieur de ces flacons, celui de leur col ainsi que leur hauteur.

Tableau 2

| Série | Contenance utile (ml) | Contenance ras-bord (ml) | Diamètre intérieur (mm) | Diamètre du col (mm) | Hauteur (mm) |
|---|---|---|---|---|---|
| 1 | 5 | 7 | 23,50 | 20,0 | 40,0 |
| 2 | 15 | 17 | 29,90 | 20,0 | 60,0 |
| 3 | 20 | 22 | 29,90 | 20,0 | 60,0 |
| 4 | 50 | 60 | 42,47 | 20,0 | 70,0 |

Ces flacons, utilisés pour la première fois, ont été préalablement soumis à trois cycles de lavage et de rinçage avec une eau chaude portée à environ 50°C et de qualité dite PPI avant d'être séchés.

Trois solutions mères d'oxaliplatine à des concentrations respectivement de 2 mg/ml, 5 mg/ml et 7 mg/ml ont été préparées de façon habituelle en utilisant de l'eau de qualité PPI comme solvant. Aucun agent stabilisant particulier n'a été utilisé.

Des aliquots de ces préparations ont été prélevés puis transvasés dans des conditions de remplissage aseptique dans les différents flacons de façon à atteindre un niveau respectif correspondant aux hauteurs indiquées ci-après.

Les flacons ont été ensuite hermétiquement fermés par sertissage d'une capsule.

En vue des études de stabilité en conditions normales, une première partie de ces flacons ont été ensuite placés dans une première enceinte thermostatée à une température de 25°C et à une humidité relative de 60%. Ces flacons ont été maintenus debout et au repos sans agitation particulière pendant les périodes indiquées ci-après.

Des échantillons ont été prélevés aux périodes indiquées puis analysés par chromatographie liquide à haute performance selon une méthode classique afin de quantifier le taux d'impuretés totales non caractérisées, exprimé en pourcentage pondéral, par rapport aux quantités d'oxaliplatine présentent dans chacun des échantillons.

2. Résultats de l'étude de la stabilité de préparations d'oxaliplatine en solution aqueuse à une concentration de 5 mg/ml

Cette étude a été conduite sur des lots de flacons d'une contenance utile respectivement de 5 ml, 15 ml et 20 ml, remplis en procédant comme décrit précédemment avec des aliquots d'une solution mère à une concentration de 5 mg/ml, puis conservés dans les conditions mentionnées précédemment pendant une période d'au moins 10 mois.

Des prélèvements d'échantillons ont été effectués respectivement à des périodes de 1 mois, puis 2,5 mois, 4 mois, 5,5 mois, 7 mois et 10 mois après la mise en flacon.

Le Tableau 3 ci-dessous rassemble, pour chacun des flacons de contenance utile respective de 5 ml, 10 ml et 15 ml, le diamètre intérieur du flacon, la hauteur de remplissage de la préparation liquide, le volume de remplissage de préparation aqueuse et la surface calculée des parois du flacon se trouvant au contact de cette préparation aqueuse puis le rapport surface/volume. Le Tableau 4 rassemble, pour chacun des flacons, le taux d'impuretés totales mesuré à un moment donné indiqué et exprimé en pourcentage pondéral par rapport à la quantité d'oxaliplatine présente.

Tableau 3

| Contenance utile (ml) | Diamètre intérieur (mm) | Hauteur de remplissage (mm) | Volume de remplissage $x10^3 \pm 4\%$ $(mm^3)$ | Surface de contact $x10^2$ $(mm^2)$ | Rapport surface/ volume |
|---|---|---|---|---|---|
| 5 | 23,50 | 10,58 | 4,59 | 12,15 | 0,26 |
| 15 | 29,90 | 15,55 | 10,92 | 21,63 | 0,20 |
| 20 | 29,90 | 30,51 | 21,42 | 35,67 | 0,17 |
| 50 | 42,47 | 35,30 | 50,00 | 61,25 | 0,12 |

Tableau 4

| Contenance utile | Taux d'impureté (% pondéral) 1 mois | Taux d'impureté 2,5 mois | Taux d'impureté 4 mois | Taux d'impureté 5,5 mois | Taux d'impureté 7 mois | Taux d'impureté 10 mois |
|---|---|---|---|---|---|---|
| 5 ml | 2,34 | 2,55 | 2,89 | 2,70 | 3,19 | 3,64 |
| 15 ml | 1,15 | 1,16 | 1,23 | 1,50 | 1,56 | 1,59 |
| 20 ml | 1,06 | 1,11 | 1,13 | 1,38 | 1,42 | 1,45 |

On remarque que le flacon de contenance utile de 5 ml ne donne pas satisfaction car le taux maximal d'impuretés totales non caractérisé admis, soit 2,0 %, est déjà dépassé lorsque l'analyse initiale effectuée 1 mois après la mise en solution.

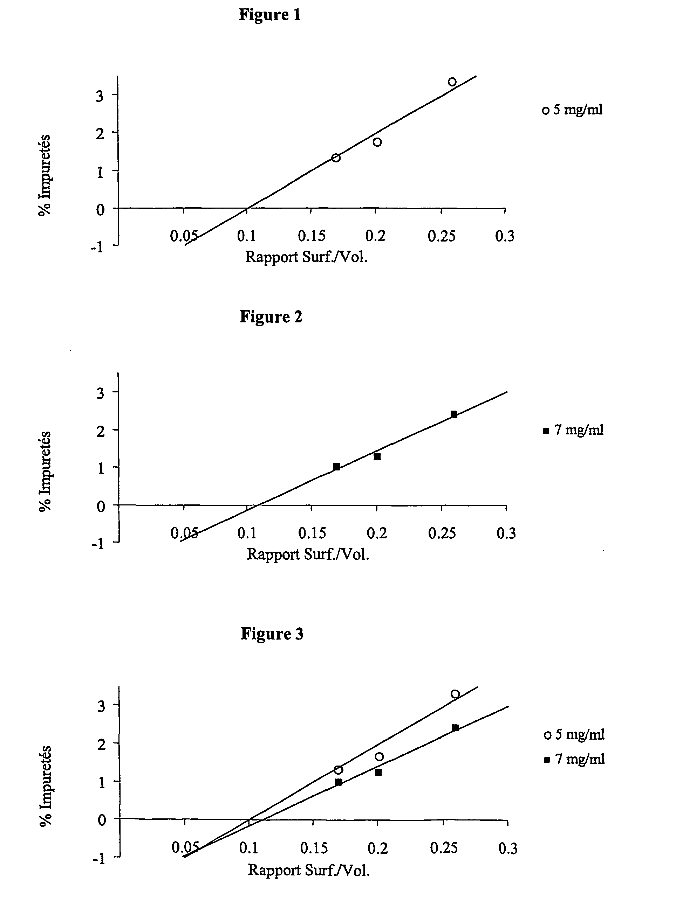

La figure 1 représente les valeurs de la colonne "4 mois" du tableau 4 en fonction du rapport Surface/Volume.

3. Résultats de l'étude de la stabilité de préparations d'oxaliplatine en solution aqueuse à une concentration de 7 mg/ml

Cette étude a été conduite comme précédemment, avec la différence que les flacons ont été chargés avec des aliquots d'une solution mère à une concentration de 7 mg/ml et des prélèvements d'échantillons ont été effectués aux mêmes périodes.

Le Tableau 5 ci-dessous rassemble, pour chacun des flacons, le taux d'impuretés totales mesuré à un moment donné indiqué et exprimé en pourcentage pondéral par rapport à la quantité d'oxaliplatine présente.

Tableau 5

| Contenance utile | Taux d'impureté (% pondéral) 1 mois | Taux d'impureté 2,5 mois | Taux d'impureté 4 mois | Taux d'impureté 5,5 mois | Taux d'impureté 7 mois | Taux d'impureté 10 mois |
|---|---|---|---|---|---|---|
| 5 ml | 1,87 | 2,09 | 2,33 | 2,56 | 2,75 | 2,98 |
| 15 ml | 0,96 | 1,03 | 1,12 | 1,19 | 1,23 | 1,30 |
| 20 ml | 0,70 | 0,81 | 0,97 | 1,04 | 1,07 | 1,11 |

A l'instar de l'essai effectué sur la préparation aqueuse d'oxaliplatine à une concentration de 5 mg/ml, on remarque également que le flacon de contenance utile de 5 ml ne donne pas satisfaction. Cependant, le taux maximal d'impuretés totales non caractérisées admis n'est dépassé qu'ultérieurement. Il en résulte que la stabilité de la solution augmente avec la concentration.

La figure 2 représente les valeurs de la colonne "4 mois" du tableau 5 en fonction du rapport Surtace/Volume.

La figure 3 représente une superposition des courbes de la figure 1 et 2, ce qui permet de mieux faire ressortir le fait que la stabilité de la solution augmente avec la concentration.

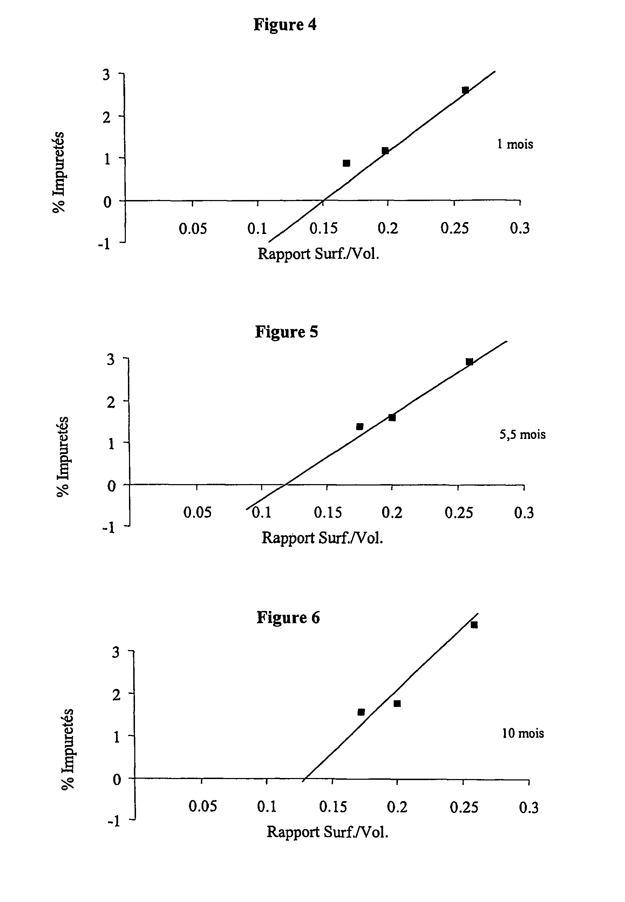

Les figures 4 à 6 représentent respectivement les valeurs des colonnes "1 mois", "5,5 mois" et "10 mois" du tableau 5 en fonction du rapport SurfaceNolume.

4. Résultats de l'étude de la stabilité à long terme de préparations d'oxaliplatine

en solution aqueuse à une concentration de 2 mg/ml

Cette étude a été conduite sur trois lots de flacons d'une même contenance utile de 50 ml, remplis en procédant comme décrit précédemment avec des aliquots d'un même volume d'une solution mère à une concentration de 2 mg/ml, puis conservés dans les conditions mentionnées précédemment pendant une période de 5 années. A l'issu de cette période, des échantillons ont été prélevés pour être analysés.

Le tableau 6 ci-dessous rassemble, pour ces flacons de même contenance utile, leur diamètre intérieur, la hauteur de remplissage de la préparation liquide, le volume de remplissage de préparation aqueuse et la surface calculée des parois du flacon se trouvant au contact de cette préparation aqueuse puis le rapport surface/volume. Le Tableau 7 rassemble, pour chacun des flacons, le taux d'impuretés totales mesuré après 5 ans.

Tableau 6

| Contenance utile (ml) | diamètre intérieur (mm) | Hauteur de remplissage (mm) | Volume de remplissage $\times 10^3 \pm 4\%$ ($mm^3$) | Surface de contact $\times 10^2$ ($mm^2$) | Rapport surface/ volume |
|---|---|---|---|---|---|
| 50 | 42,47 | 35,30 | 50,00 | 61,25 | 0,12 |

Tableau 7

| Lot | Taux d'impureté (% pondéral) 5 ans |
|---|---|
| 1 | 1,47 |
| 2 | 1,56 |
| 3 | 1,55 |

5. Commentaires et conclusions

A partir de l'enseignement des figures 1 et 2, on remarque que le taux d'impuretés totales non caractérisées diminue lorsque le rapport SurfaceNolume diminue.

Même dès le début du stockage des flacons d'oxaliplatine, la stabilité de la solution est meilleure pour rapport SurfaceNolume faible.

En outre, on observe une relation linéaire entre le rapport Surface/Volume et le taux d'impuretés.

En tenant compte des résultats présentés ci-dessus, l'équation générale suivante peut être déduite:

$$R = R_0 + A.c.I$$

où

I      représente le taux d'impuretés totales non caractérisées présentes dans la préparation aqueuse d'oxaliplatine à concentration donnée;

$R_0$      représente le rapport SurfaceNolume maximal théorique pour lequel aucune impureté ne serait quantifiable (c'est à dire pour I = 0) en mettant en oeuvre techniques d'analyse habituellement recommandée par la pharmacopée, cette valeur étant fonction de la concentration en oxaliplatine dans la préparation;

A      est une constante exprimée en ml/(mg.mm);

c      représente la concentration en oxaliplatine dans la préparation exprimée en mg/ml; et

R      représente le rapport Surface/Volume propre au flacon considéré à un remplissage donné.

En prenant les résultats illustrés sur la figure 1, on peut déduire de la courbe les valeurs suivantes:
A = 0,01 ml/(mg,m) et $R_0$= 0,10 pour c = 5 mg/ml

En prenant les résultats illustrés sur la figure 2, on peut déduire de la courbe les valeurs suivantes:
A = 0,009 ml/(mg.m) et $R_0$ = 0,11 pour c = 5 mg/ml

A noter par ailleurs, comme on peut le constater sur les figures 4 à 6, que la stabilité de la préparation diminue au cours du temps de façon linéaire.

Ainsi devient-il possible de choisir un rapport Surface/Volume approprié, par exemple 0,1, lorsque l'on fixe un temps de conservation donné, par exemple 3 ans.

Pratiquement, pour déterminer le rapport SurfaceNolume à ne pas dépasser pour un flaconnage donné contenant une préparation pharmaceutique d'oxaliplatine en solution aqueuse à une concentration donnée, on peut procéder de la manière suivante :

On utilise au moins deux flacons de forme similaire mais de volumes différents (donc de rapports SurfaceNolume différents) que l'on remplis avec la préparation aqueuse d'oxaliplatine.

On détermine alors les rapports Surface/Volume puis on quantifie les taux d'impuretés totales non caractérisées respectifs à des durées de conservation données (par exemple à 1 mois ou 4 mois). On établit alors un graphe dans lequel on reporte les taux d'impuretés mesurés en fonction du rapport "Surface/Volume" et on détermine l'endroit où l'abscisse et la courbe se croisent. La valeur ainsi obtenue donne le rapport SurfaceNolume qu'il ne faudrait pas dépasser.

La Demanderesse a outre constaté que cette invention est particulièrement efficace pour un volume de remplissage supérieur à 7 ml. De préférence, la présente invention est applicable à toute solution d'oxaliplatine contenue dans un flacon de contenance utile égale ou supérieure à 10 ml.

De préférence, les préparations pharmaceutiques d'oxaliplatine conservées sont celles dans lesquelles l'oxaliplatine se trouve en solution aqueuse à des concentrations comprises entre 2 et 7 mg/ml.

On notera enfin que la Demanderesse a réalisé une étude de stabilité en conditions accélérées destinée à anticiper des stabilités à l'échelle de 3 ans. A cet effet, des flacons ont été placés dans une enceinte thermostatée à une température de 40°C et dans une atmosphère humidifiée à 75%. Des prélèvements ont été régulièrement effectués puis analysés.

Les résultats obtenus laissent apparaître que des préparations pharmaceutiques d'oxaliplatine en solution aqueuse pourrons être conservées dans les flacons déjà retenus précédemment et dans les conditions de remplissage indiquées pendant une période allant au moins jusqu'à 36 mois, satisfaisant ainsi aux meilleures périodes de conservation reconnues par les autorités de santé.

Cependant, il va sans dire que le spécialiste saura appliquer l'invention sans se limiter ni aux concentrations utilisées, ni aux formes des flacons (flacons à base parallélépipédique ou cylindrique) ou aux types de verre utilisés dans les exemples précédents. En outre, l'invention est applicable à toute préparation pharmaceutique d'oxaliplatine en solution aqueuse, celle-ci pouvant contenir en outre des éléments comme des agents de stabilisation (p.ex. des agents tampons).

# EP 1 368 022 B1

**Revendications**

1. Utilisation d'un flacon en verre contenant une préparation pharmaceutique d'oxaliplatine en solution aqueuse à une concentration comprise entre 2mg/ml et 7mg/ml, flacon dont le rapport SurfaceNolume, exprimé en mm²/mm³, est inférieur à 0,20 et dont la contenance utile est égale ou supérieure à 10 ml, pour maintenir sur une durée d'au moins 10 mois ladite préparation avec un taux d'impuretés total ne dépassant pas 2 % en poids par rapport au poids d'oxaliplatine, ladite Surface étant la surface de contact de la solution aqueuse d'oxaliplatine avec le flacon et ledit Volume étant le volume de remplissage du flacon par ladite solution.

2. Utilisation selon la revendication 1, dans laquelle la concentration est de 2mg/ml.

3. Utilisation selon la revendication 1, dans laquelle la concentration est de 5mg/ml.

4. Utilisation selon l'une des revendications précédentes dans laquelle ledit flacon est en verre de type I.


**Claims**

1. Use of a glass bottle containing a pharmaceutical preparation of oxaliplatin in aqueous solution at a concentration comprised between 2 mg/ml and 7 mg/ml, the bottle having a surface/volume ratio, expressed in mm²/mm³, of less than 0.20 and which useful capacity is equal to or greater than 10 ml; to maintain during at least 10 months said preparation with a total level of impurities not exceeding 2% by weight relative to the weight of the oxaliplatin, said surface being the contact surface of the aqueous oxaliplatin solution with the bottle and said volume being the bottle volume filled with said solution.

2. Use as claimed in claim 1, in which the concentration is 2 mg/ml.

3. Use as claimed in claim 1, in which the concentration is 5 mg/ml.

4. Use as claimed in any one of the preceding claims, in which said bottle is made of glass of type I.


**Patentansprüche**

1. Verwendung einer Glasflasche die einer pharmazeutischen Oxaliplatin-Zubereitung in wäßriger Lösung in einer Konzentration zwischen 2 mg/ml und 7 mg/ml enthält, Flasche mit einem Oberfläche/Volumen-Verhältnis, ausgedrückt in mm²/mm³, kleiner als 0,20 ist, und mit einem Nutzinhalt größer gleich 10 ml ist, um die genannte Zubereitung mit einem Gesamtgehalt an Verunreinigungen über einen Zeitraum von mindestens 10 Monaten nicht über 2,0 Gew.-%, bezogen auf das Gewicht des Oxaliplatins, liegen; die genannte "Oberfläche" bezeichnet die Oberfläche des Kontakts der wäßrigen Oxaliplatin-Lösung mit der Flasche und das genannte "Volumen" bezeichnet das Füllvolumen der Oxaliplatin-Lösung.

2. Verwendung nach Anspruch 1, in der die Konzentration 2 mg/ml ist.

3. Verwendung nach Anspruch 1, in der die Konzentration 5 mg/ml ist.

4. Verwendung nach einem der vorhergehenden Ansprüche, in der die Flasche aus Glas vom Typ I ist.

**Figure 1**

o 5 mg/ml

**Figure 2**

■ 7 mg/ml

**Figure 3**

o 5 mg/ml

■ 7 mg/ml

## Figure 4

## Figure 5

## Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9604904 A, Ibrahim
- WO 0021527 A
- WO 9943355 A

**Littérature non-brevet citée dans la description**

- **KIDANI et al.** *J. Med. Chem.,* 1978, vol. 21, 1315
- The Merck Index. 1989, 1093
- *United States Pharmacopeia,* 2002, vol. 25, 20
- Pharmacopée Européenne. 2002
- Technical Methods Bulletin. *Glass containers for small volume parenteral products: Factors for selection and test methods for identification, Parenteral drug association,* 1982